# EUROPEAN PATENT SPECIFICATION

(11) **EP 2 347 713 B1**
(45) Date of publication and mention of the grant of the patent: **03.03.2021**
(21) Application number: 11151475.8
(22) Date of filing: 20.01.2011
(51) Int. Cl.: A61B 8/00, G01S 15/89

(54) **Ultrasound image enhancement in an ultrasound system**
Ultraschallbildverbesserung in einem Ultraschallsystem
Amélioration de l'image à ultrasons dans un système à ultrasons

(30) Priority: 25.01.2010 KR 20100006412
(43) Date of publication of application: 27.07.2011
(73) Proprietor: Samsung Medison Co., Ltd., Hongcheon-gun, Gangwon-do, 25108 (KR)
(72) Inventor: Yoo, Jae Heung, 135-851, Seoul (KR); Kim, Jeong Sik, 135-851, Seoul (KR)
(74) Representative: Schmid, Wolfgang

(56) References cited:
- WO-A1-00/20889
- US-B1- 6 181 810
- US-B2- 6 895 077
- MYOUNG HWAN CHOI ED - CELLER B G ET AL: "Suppression of Gradient Across Seam Line Using a Smoothing Filter in Spatially Compounded Ultrasonic Diagnostic Images", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 August 2007 (2007-08-22), pages 2142-2145, XP031336625, ISBN: 978-1-4244-0787-3
- MH CHOI ET AL: "Method for suppressing seam line artifact in spatially compounded ultrasonic diagnostic images", PROCEEDINGS OF THE FIFTH IASTED INTERNATIONAL CONFERENCE ON BIOMEDICAL ENGINEERING : FEBRUARY 14 - 16, 2007, INNSBRUCK, AUSTRIA / ED.: J. W. GARDNER,, 1 January 2007 (2007-01-01), pages 192-196, XP008136712, ISBN: 978-0-88986-648-5
- YINGEN XIONG AND KARI PULLI: "Mask-based Image Blending and its Applications on Mobile", MIPPR 2009, PROC. OF SPIE, vol. 7498, 2009, XP040503795,

## Description

### CROSS-REFERENCE TO RELATED APPLICATIONS

The present application claims priority from Korean Patent Application No. 10-2010-0006412 filed on January 25, 2010.

### TECHNICAL FIELD

The present disclosure generally relates to ultrasound image processing, and more particularly to enhancing ultrasound images without lowering a frame rate in an ultrasound system.

### BACKGROUND

An ultrasound system has been extensively used in the medical field due to its non-invasive and non-destructive nature. Modern high-performance ultrasound imaging diagnostic systems and techniques are commonly used to produce two-dimensional or three-dimensional ultrasound images of internal features of patients.

Recently, spatial compounding has been adopted in the ultrasound system to provide enhanced ultrasound images. The spatial compounding is implemented by compounding a plurality of ultrasound images (e.g., three ultrasound images), which have been successively formed at different steering angles of scan lines, to form a compound image. In such a case, when the ultrasound images are acquired under the condition that a surface of an ultrasound probe is not properly contacted with a surface of a target object, border lines of the ultrasound images may appear in a compound image formed by spatial compounding of the ultrasound images, i.e., seam artifact occurs in the compound image, which may degrade the compound image.

An ultrasound system according to the preamble of claim 1 and a method of forming an ultrasound spatial compound image in this ultrasound system is known from XP031336625 "Suppression of Gradient Across Seam Line Using a Smoothing Filter in Spatially Compounded Ultrasonic Diagnostic Images" by Myoung Hwan Choi.

### SUMMARY

Embodiments for spatially compounding ultrasound images for removing seam artifact in an ultrasound system are disclosed herein. In one embodiment, by way of nonlimiting example, an ultrasound system comprises the features according to claim 1.

In another embodiment, a method of forming an ultrasound spatial compound image in an ultrasound system, comprises the features according to claim 4.

Furthermore, the invention relates to a computer-readable storage medium according to claim 7.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit of FIG. 1.
- FIG. 3: is a schematic diagram showing examples of frames consisting of a scan line group with its scan lines not being steered and scan line groups with their scan lines being steered at predetermined steering angles θ₁ and θ₂. lines being steered at predetermined steering angles θ₁ and θ₂.
- FIG. 4: is a block diagram showing an illustrative embodiment of a processing unit of FIG. 1.
- FIG. 5: is a schematic diagram showing examples of masks.
- FIG. 6: is a schematic diagram showing examples of mask images, ultrasound image and a compound image.

### DETAILED DESCRIPTION

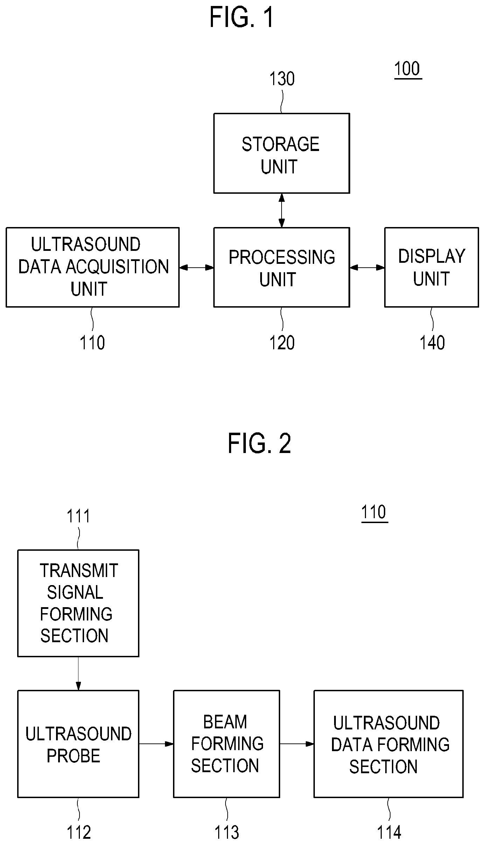

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure. Referring to FIG. 1, an ultrasound system constructed in accordance with one embodiment is shown. The ultrasound system 100 includes an ultrasound data acquisition unit 110, a processing unit 120, a storage unit 130 and a display unit 140. The ultrasound data acquisition unit 110 is configured to transmit ultrasound beams to a target object and receive ultrasound echoes reflected from the target object to thereby form ultrasound data representative of the target object. An operation of the ultrasound acquisition unit will be described in detail by referring to FIG. 2.

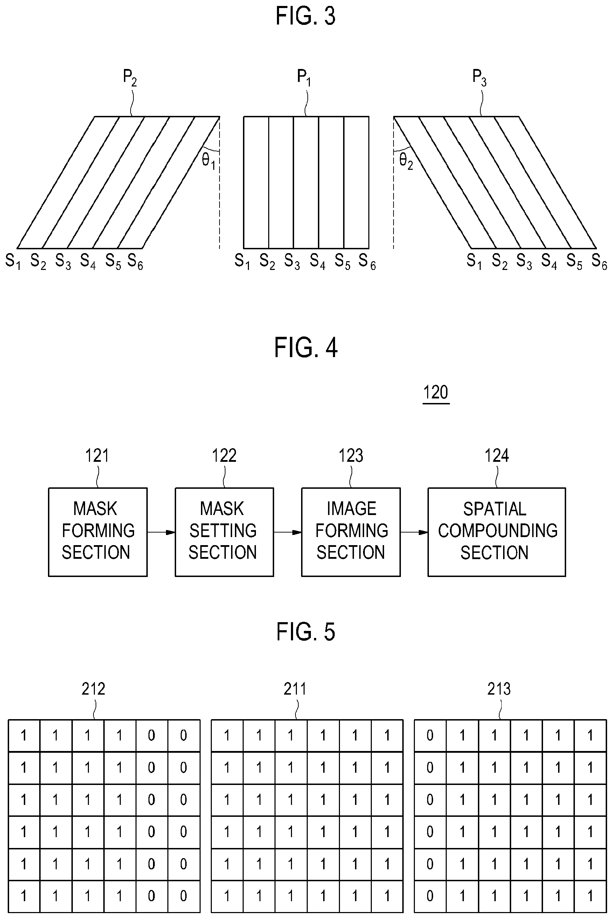

FIG. 2 is a block diagram showing an illustrative embodiment of the ultrasound data acquisition unit 120. Referring to FIG. 2, the ultrasound data acquisition unit 110 includes a transmit (Tx) signal forming section 111. The Tx signal forming section 121 generates a plurality of Tx signals and apply delays to the Tx signals. The delays of the Tx signals is controlled according to a steering angle of scan lines. For example, the Tx signals includes first Tx signals for obtaining a first frame P₁ in which scan lines S₁-S₆ are not steered, second Tx signals for obtaining a second frame P₂ in which scan lines S₁-S₆ are steered at a first steering angle of θ₁ and third Tx signals for obtaining a third frame P₃ in which scan lines S₁-S₆ are steered at a second steering angle of θ₂, as shown in FIG. 3.

The ultrasound data acquisition unit 110 further includes an ultrasound probe 112, which is coupled to the Tx signal forming section 111. The ultrasound probe 112 includes an array transducer containing a plurality of transducer elements for reciprocal conversion between electric signals and ultrasound signals. The ultrasound probe 112 is configured to transmit ultrasound signals in response to the Tx signals. The ultrasound probe 112 is further configured to receive ultrasound echoes reflected from the target object to thereby output receive signals. In one embodiment, the receive signals includes first receive signals obtained in response to the first Tx signals, second receive signals obtained in response to the second Tx signals and third receive signals obtained in response to the third Tx signals.

The ultrasound data acquisition unit 110 further includes a beam forming section 113, which is coupled to the ultrasound probe 112. The beam forming section 113 is configured to digitize the electrical receive signals to obtain digital signals. The beam forming section 113 also applies delays to the digital signals in consideration of distances between the elements of the ultrasound probe 112 and focal points. The beam forming section 113 further sums the delayed digital signals to form receive-focused beams. In one embodiment, the beam forming section 113 forms a first receive-focused beam based on the first receive signals, a second receive-focused beam based on the second receive signals and a third receive-focused beam based on the third receive signals.

The ultrasound data acquisition unit 110 further includes an ultrasound data forming section 114, which is coupled to the beam forming section 113. The ultrasound data forming section 114 is configured to form ultrasound data corresponding to a plurality of frames based on the receive-focused beams. The ultrasound data are stored in the storage unit 130. In one embodiment, the ultrasound data forming section 114 is configured to form a first ultrasound frame data set corresponding to the respective scan lines S₁-S₆ of the first frame P₁ based on the first receive-focused beams. The ultrasound data forming section 114 is configured to form a second ultrasound frame data set corresponding to the respective scan lines S₁-S₆ of the second frame P₂ based on the second receive-focused beams. Further, the ultrasound data forming section 114 is configured to form a third ultrasound frame data set corresponding to the respective scan lines S₁-S₆ of the third frame P₃ based on the first receive-focused beams. The ultrasound data forming section 114 is configured to perform a variety of signal processing (e.g., gain adjustment, etc.), which is necessary for forming the ultrasound data, upon the receive-focused beams.

Referring back to FIG. 1, the processing unit 120, which is coupled to the ultrasound data acquisition unit 110, is configured to form ultrasound images and mask images corresponding to the frames of the plurality of steering angles based on the plurality of ultrasound frame data sets. In this case, the mask images are formed to remove seam artifacts appearing in a spatial compound image of the ultrasound images. Further, the processing unit 120 is configured to spatially compound a plurality ultrasound images by using the mask images to from an ultrasound spatial compound image. An operation of the processing unit 120 will be described in detail by referring to FIG. 4. FIG. 4 is a block diagram showing an illustrative embodiment of the processing unit 120. In one embodiment, the processing unit 120 includes a mask forming section 121, a mask setting section 122, an image forming section 123 and a spatial compounding section 124.

The mask forming section 121 is configured to a plurality of masks corresponding to a plurality of frames, respectively, based on the plurality of ultrasound frame data sets. Each of the masks has a size and a pixel number identical to those of each of the frames. In one embodiment, the mask forming section 121 is configured to a first mask 211 corresponding to the first frame P₁ based on the first ultrasound frame data set as shown in FIG. 5. The mask forming section 121 is configured to a second mask 212 corresponding to the first frame P₂ based on the second ultrasound frame data set. Further, the mask forming section 121 is configured to a third mask 213 corresponding to the first frame P₃ based on the third ultrasound frame data set.

The mask setting section 122, which is coupled to the mask forming section 121, is configured to determine a pixel value corresponding to each of pixels in each of the masks by using the ultrasound frame data sets. More particularly, the mask setting section 122 is configured to detect an intensity of the ultrasound frame data corresponding to the scan line Sᵢ at each of the frame by using the ultrasound frame data sets. The mask setting section 122 is further configured to compare the detected intensity with a predetermined threshold. If the intensity is equal to or greater than the predetermined threshold, then it is determined that the ultrasound probe 122 is properly contacted with the surface of the target object. This is so that the mask setting section 122 may assign a value of 1 to pixels corresponding to the scan line Sᵢ.

On the other hand, if the intensity is less than the predetermined threshold, then it is determined that the ultrasound 122 is properly contracted with the surface of the target object. This is so that the mask setting section 122 may assign a value of 0 to pixels corresponding to the scan line Sᵢ. For example, the mask setting section 122 is configured to detect intensities of the ultrasound frame data corresponding to each of the scan lines S₁-S₆ of the first frame P₁ and compare the intensities with the predetermined threshold to thereby assign a value of 1 to pixels corresponding to the scan line S₁-S₆, which have the intensities equal to or greater than the predetermined threshold value, as shown in FIG. 5. The mask setting section 122 is configured to detect intensities of the ultrasound frame data corresponding to each of the scan lines S₁-S₆ of the second frame P₂ and compare the intensities with the predetermined threshold. The mask setting section 122 is configured to assign a value of 1 to pixels of the second mask 212 corresponding to the scan line S₁-S₄, which have the intensities equal to or greater than the predetermined threshold value, and a value of 0 to pixels of the second mask 212 corresponding to the scan line S₅-S₆, which have the intensities less than the predetermined threshold value. The mask setting section 122 is configured to detect intensities of the ultrasound frame data corresponding to each of the scan lines S₁-S₆ of the third frame P₃ and compare the intensities with the predetermined threshold. The mask setting section 122 is configured to assign a value of 1 to pixels of the third mask 213 corresponding to the scan line S₂-S₆, which have the intensities equal to or greater than the predetermined threshold value, and a value of 0 to pixels of the third mask 213 corresponding to the scan line S₁, which have the intensities less than the predetermined threshold value.

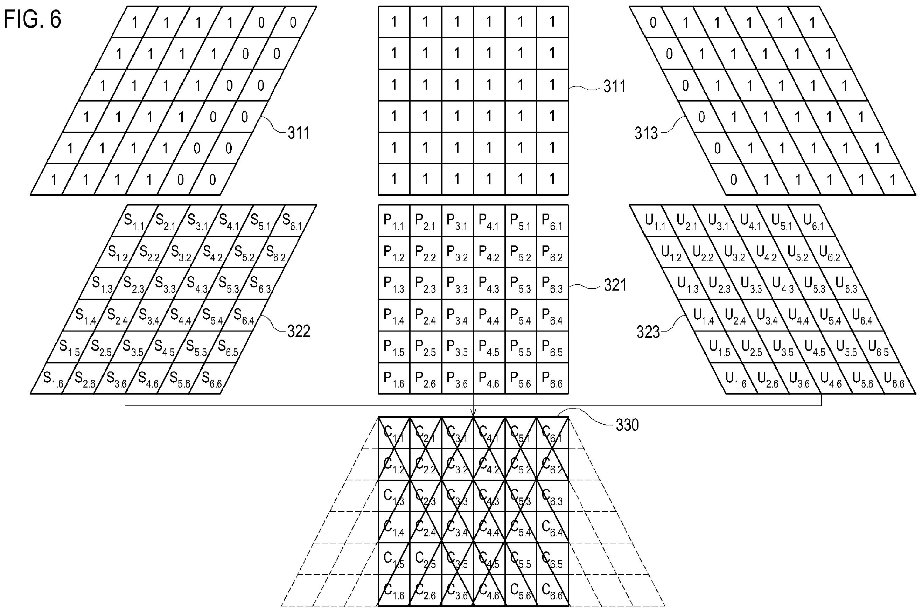

The image forming section 123, which is coupled to the mask setting unit 122, is configured to form a plurality of mask images based on the plurality of masks provided from the mask setting unit 122. Also, the image forming section 123 is further configured to form a plurality of ultrasound images corresponding to the plurality of mask images by using the ultrasound frame data sets provided from the ultrasound data acquisition unit 110. In one embodiment, the image forming section 123 is configured to form a first mask image 311, a second mask image 312 and a third mask image 313 by using the first mask 211, the second mask 212 and the third mask 213, as shown in FIG. 6. Further, the image forming section 123 is configured to form first to third ultrasound images 321 to 323 corresponding to the first to third frames P₁-P₃ by using the first to third ultrasound frame data sets.

The spatial compounding section 124 is configured to form an ultrasound spatial compound image by compounding the plurality of ultrasound images by using the plurality of mask images. The spatial compounding section 124 is configured to sum values of pixels identically positioned in the plurality of ultrasound images to obtain first summation values, and sum values of pixels identically positioned in the plurality of mask images to obtain second summation values. The spatial compound section 124 is configured to determine pixel values of the ultrasound spatial compound image. In one embodiment, the spatial compound image 124 is configured to sum a pixel value of a pixel P_{1,1} of the first ultrasound image 321, a pixel value of a pixel S_{1,1} of the second ultrasound image 322 and a pixel value of a pixel U_{1,1} of the third ultrasound image 323, with respect to a pixel C_{1,1} of a ultrasound spatial compound image 330, thereby obtaining a first summation value. The spatial compound image 124 is further configured to sum a pixel value of 1 of the first mask image 311, which corresponds to the pixel P_{1,1} of the first ultrasound image 321, a pixel value of 1 of the second mask image 312, which corresponds to the pixel S_{1,1} of the second ultrasound image, and a pixel value of 0 of the third mask image 313, which corresponds to the pixel U_{1,1} of the third ultrasound image, thereby obtaining a second summation value of 2. The spatial compounding section 124 is further configured to divide the first summation value by the second summation value (i.e., first summation value/second summation value) to thereby determine a pixel value of a pixel C_{1,1} of the ultrasound spatial compound image 330. In the same manner, the spatial compounding section 124 is configured to determine pixel values of pixels C1,2 and C_{1,3} of the ultrasound spatial compound image 330.

With respect to a pixel C_{1,4} of the ultrasound spatial compound image 330, the spatial compounding section 124 is configured to sum a pixel value of a pixel P_{1,4} of the first ultrasound image 321 and a pixel value of a pixel S_{2,4} of the second ultrasound image 322, thereby obtaining a first summation value. The spatial compound image 124 is further configured to sum a pixel value of 1 of the first mask image 311, which corresponds to the pixel P_{1,4} of the first ultrasound image 321, and a pixel value of 1 of the second mask image 312, which corresponds to the pixel S_{2,4} of the second ultrasound image, thereby obtaining a second summation value of 2. The spatial compounding section 124 is further configured to divide the first summation value by the second summation value (i.e., first summation value/second summation value) to thereby determine a pixel value of a pixel C_{1,4} of the ultrasound spatial compound image 330. In the same manner, the spatial compounding section 124 is configured to determine pixel values of pixels C_{1,5} and C_{1,6} of the ultrasound spatial compound image 330.

With respect to a pixel C_{2,1} of the ultrasound spatial compound image 330, the spatial compound image 124 is configured to sum a pixel value of a pixel P_{2,1} of the first ultrasound image 321, a pixel value of a pixel S2,1 of the second ultrasound image 322 and a pixel value of a pixel U_{2,1} of the third ultrasound image 323, thereby obtaining a first summation value. The spatial compound image 124 is further configured to sum a pixel value of 1 of the first mask image 311, which corresponds to the pixel P_{2,1} of the first ultrasound image 321, a pixel value of 1 of the second mask image 312, which corresponds to the pixel S_{2,1} of the second ultrasound image, and a pixel value of 1 of the third mask image 313, which corresponds to the pixel U_{2,1} of the third ultrasound image, thereby obtaining a second summation value of 3. The spatial compounding section 124 is further configured to divide the first summation value by the second summation value (i.e., first summation value/second summation value) to thereby determine a pixel value of a pixel C_{2,1} of the ultrasound spatial compound image 330. In the same manner, the spatial compounding section 124 is configured to determine pixel values of pixels C_{2,2}, C_{2,3}, C_{2,4}, C_{2,5}, C_{2,6}, C_{3,1}, C_{3,2}, C_{3,3}, C_{3,4}, C_{3,5}, C_{3,6}, C_{4,1}, C_{4,2}, C_{4,3}, C_{4,4}, C_{4,5}, C_{4,6}, C_{5,1}, C_{5,2}, C_{5,3}, C_{5,4}, C_{5,5}, C_{5,6}, C_{6,1}, C_{6,2} and C_{6,3} of the ultrasound spatial compound image 330.

With respect to a pixel C_{6,4} of the ultrasound spatial compound image 330, the spatial compounding section 124 is configured to sum a pixel value of a pixel P_{6,4} of the first ultrasound image 321 and a pixel value of a pixel U_{5,4} of the second ultrasound image 323, thereby obtaining a first summation value. The spatial compound image 124 is further configured to sum a pixel value of 1 of the first mask image 311, which corresponds to the pixel P_{6,4} of the first ultrasound image 321, and a pixel value of 1 of the third mask image 313, which corresponds to the pixel U_{5,4} of the third ultrasound image, thereby obtaining a second summation value of 2. The spatial compounding section 124 is further configured to divide the first summation value by the second summation value (i.e., first summation value/second summation value) to thereby determine a pixel value of a pixel C_{6,4} of the ultrasound spatial compound image 330. In the same manner, the spatial compounding section 124 is configured to determine pixel values of pixels C_{6,5} and C_{6,6} of the ultrasound spatial compound image 330. Referring back to FIG. 1, the storage unit 130, which is coupled to the processing unit 120, is configured to store the ultrasound data sets, which have been acquired in the ultrasound data acquisition unit 110. The storage unit 130 further stores the plurality of ultrasound images, which have been formed in the processing unit 120. The display unit 140 displays the ultrasound spatial compound image.

In another embodiment, there is provided a computer-readable storage medium storing instructions that, when executed by a computer, cause the computer to provide a method of spatially compounding ultrasound images based on a plurality of ultrasound frame data sets acquired from a target object and at different steering angles of scan lines in an ultrasound system, the method comprising: setting a plurality of masks corresponding to the respective frames based on the plurality of ultrasound frame date sets for removing seam artifact to form a plurality of mask images corresponding to the respective frames; forming a plurality of ultrasound images corresponding to the plurality of frames based on the plurality of frame data sets; and spatially compounding the plurality of ultrasound image based on the plurality of mask images to form an ultrasound spatial compound image.

Although embodiments have been described with reference to a number of illustrative embodiments thereof, it should be understood that numerous other modifications and embodiments can be devised by those skilled in the art. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system (100), comprising:
an ultrasound data acquisition unit (110) configured to transmit ultrasound beams to a target object, receive ultrasound echoes reflected from the target object and provide a plurality of ultrasound frame data sets for a plurality of frames, said plurality of ultrasound frame data being acquired at different steering angles of scan lines; and
a processing unit (120) configured to form a plurality of ultrasound images,
wherein the processing unit (120) includes:
a mask forming section configured to form masks corresponding to the respective frames based on the ultrasound frame data sets,
a mask setting section configured to determine pixel values of pixels of each of the mask based on intensities of the ultrasound frame data sets,
an image forming section configured to form a plurality of mask images based on the masks and the plurality of ultrasound images based on the ultrasound frame data sets, and
a spatially compounding section configured to spatially compound the plurality of ultrasound image based on the plurality of mask images to form an ultrasound spatial compound image,
wherein the spatial compounding section is configured to sum pixel values of pixels overlapped at the ultrasound images to obtain a first summation value, sum pixel values of pixels overlapped at the mask images to obtain a second summation value and divide the first summation value by the second summation value to determine a pixel value at a corresponding pixel of the ultrasound spatial compound image.

2. The ultrasound system (100) of Claim 1, wherein the mask images have identical sizes and pixel numbers to those of the corresponding ultrasound images.

3. The ultrasound system (100) of Claim 1, wherein the mask setting section is configured to detect an intensity of each of the frames corresponding to the respective scan lines by using the ultrasound frame data sets, compare the detected intensity with a predetermined threshold, assign, when the intensity is equal to or greater than a predetermined threshold, a value of 1 to pixels corresponding to the scan line, and assign, when the intensity is less than the predetermined threshold, a value of 0 to pixels corresponding to the scan line.

4. A method of forming an ultrasound spatial compound image in an ultrasound system (100), comprising:
a) transmitting ultrasound beams to a target object, receiving ultrasound echoes reflected from the target object and providing a plurality of ultrasound frame data sets for a plurality of frames, said plurality of ultrasound frame data being acquired at different steering angles of scan lines;
b) forming masks corresponding to the respective frames based on the ultrasound frame data sets;
c) determining pixel values of pixels of each of the mask based on intensities of the ultrasound frame date sets for removing seam artifact to form a plurality of mask images corresponding to the respective frames;
d) forming a plurality of ultrasound images corresponding to the plurality of frames based on the plurality of frame data sets; and
e) spatially compounding the plurality of ultrasound image based on the plurality of mask images to form an ultrasound spatial compound image,
wherein the e) includes:
summing pixel values of pixels overlapped at the ultrasound images to obtain a first summation value;
summing pixel values of pixels overlapped at the mask images to obtain a second summation value; and
dividing the first summation value by the second summation value to determine a pixel value at a corresponding pixel of the ultrasound spatial compound image.

5. The method of Claim 4, wherein the masks have identical sizes and pixel numbers to those of the corresponding ultrasound images.

6. The method of Claim 4, wherein the c) includes:
detecting an intensity of each of the frames corresponding to the respective scan lines by using the ultrasound frame data sets;
comparing the detected intensity with a predetermined threshold,
assigning, when the intensity is equal to or greater than a predetermined threshold, a value of 1 to pixels corresponding to the scan line; and
assigning, when the intensity is less than the predetermined threshold, a value of 0 to pixels corresponding to the scan line.

7. A computer-readable storage medium storing instructions that, when executed by a computer, cause the computer to provide a method of spatially compounding ultrasound images based on a plurality of ultrasound frame data sets acquired from a target object and at different steering angles of scan lines in an ultrasound system (100), the method comprising:
forming masks corresponding to the respective frames based on the ultrasound frame data sets;
determining pixel values of pixels of each of the mask based on intensities of the ultrasound frame date sets for removing seam artifact to form a plurality of mask images corresponding to the respective frames;
forming a plurality of ultrasound images corresponding to the plurality of frames based on the plurality of frame data sets; and
spatially compounding the plurality of ultrasound image based on the plurality of mask images to form an ultrasound spatial compound image,
wherein the determining pixel values of pixels of each of the mask includes:
detecting an intensity of each of the frames corresponding to the respective scan lines by using the ultrasound frame data sets;
comparing the detected intensity with a predetermined threshold, assigning, when the intensity is equal to or greater than a predetermined threshold, a value of 1 to pixels corresponding to the scan line; and
assigning, when the intensity is less than the predetermined threshold, a value of 0 to pixels corresponding to the scan line,
wherein the spatially compounding the plurality of ultrasound image includes:
summing pixel values of pixels overlapped at the ultrasound images to obtain a first summation value;
summing pixel values of pixels overlapped at the mask images to obtain a second summation value; and
dividing the first summation value by the second summation value to determine a pixel value at a corresponding pixel of the ultrasound spatial compound image.

## Patentansprüche

1. Ultraschallsystem (100), welches Folgendes aufweist:
eine Ultraschalldatenerlangungseinheit (110), die dafür vorgesehen ist, Ultraschallstrahlen zu einem Zielobjekt zu übertragen, von dem Zielobjekt reflektierte Ultraschallechos zu empfangen und eine Vielzahl von Ultraschallrahmendatensätze für eine Vielzahl von Rahmen zur Verfügung zu stellen, wobei die Vielzahl von Ultraschallrahmendaten an unterschiedlichen Steuerungswinkeln von Scanlinien erlangt werden; und
eine Verarbeitungseinheit (120), die dafür vorgesehen ist, eine Vielzahl von Ultraschallabbildungen zu erzeugen,
wobei die Verarbeitungseinheit (120) Folgendes aufweist:
einen Maskenerzeugungsabschnitt, der dafür vorgesehen ist, dem jeweiligen Rahmen entsprechende Masken basierend auf den Ultraschallrahmendatensätzen zu erzeugen,
einen Maskenfestlegungsabschnitt, der dafür vorgesehen ist, Pixelwerte von Pixeln von jeder der Masken basierend auf Intensitäten der Ultraschallrahmendatensätze zu ermitteln,
einen Abbildungserzeugungsabschnitt, der dafür vorgesehen ist, eine Vielzahl von Maskenabbildungen basierend auf den Masken und die Vielzahl von Ultraschallabbildungen basierend auf den Ultraschallrahmendatensätzen zu erzeugen, und
einen räumlichen Zusammensetzungsabschnitt, der dafür vorgesehen ist, die Vielzahl von Ultraschallabbildungen basierend auf der Vielzahl von Maskenabbildungen räumlich zusammenzusetzen, um ein räumliches, zusammengesetztes Ultraschallbild zu erzeugen,
wobei der räumliche Zusammensetzungsabschnitt dafür vorgesehen ist, Pixelwerte von an den Ultraschallabbildungen überlappten Pixeln zu summieren, um einen ersten Additionswert zu erlangen, Pixelwerte von an den Maskenabbildungen überlappten Pixeln zu summieren, um einen zweiten Additionswert zu erlangen, und den ersten Additionswert durch den zweiten Additionswert zu teilen, um einen Pixelwert an einem korrespondierenden Pixel der räumlichen zusammengesetzten Ultraschallabbildung zu ermitteln.

2. Ultraschallsystem (100) nach Anspruch 1, wobei die Maskenabbildungen identische Größen und Pixelanzahlen wie diejenigen der entsprechenden Ultraschallabbildungen aufweisen.

3. Ultraschallsystem (100) nach Anspruch 1, wobei der Maskenfestlegungsabschnitt dafür vorgesehen ist, eine Intensität von jedem der Rahmen, die den jeweiligen Scanlinien entsprechen, durch Verwenden der Ultraschallrahmendatensätze zu detektieren, die detektierten Intensitäten mit einem vorbestimmten Grenzwert zu vergleichen, wenn die Intensität gleich oder größer als ein vorbestimmter Grenzwert ist, den Pixeln, die der Scanlinie entsprechen, einen Wert von 1 zuzuordnen, und, wenn die Intensität geringer ist als der vorbestimmte Grenzwert, den Pixeln, die der Scanlinie entsprechen, einen Wert von 0 zuzuordnen.

4. Verfahren zum Erzeugen einer räumlichen, zusammengesetzten Ultraschallabbildung in einem Ultraschallsystem (100), welches Folgendes aufweist:
a) Übertragen von Ultraschallstrahlen zu einem Zielobjekt, Empfangen von Ultraschallechos, die von dem Zielobjekt reflektiert wurden, und zur Verfügung Stellen einer Vielzahl von Ultraschallrahmendatensätzen für eine Vielzahl von Rahmen, wobei die Vielzahl von Ultraschallrahmendaten an unterschiedlichen Steuerungswinkeln von Scanlinien erlangt werden;
b) Erzeugen von Masken, welche den jeweiligen Rahmen entsprechen, basierend auf den Ultraschallrahmendatensätzen;
c) Ermitteln von Pixelwerten von Pixeln von jeder der Maske basierend auf Intensitäten der Ultraschallrahmendatensätze zum Entfernen von Randartifakten, um eine Vielzahl von Maskenabbildungen zu erzeugen, die den jeweiligen Rahmen entsprechen;
d) Erzeugen einer Vielzahl von Ultraschallabbildungen, welche der Vielzahl von Rahmen entsprechen, basierend auf der Vielzahl von Rahmendatensätzen; und
e) räumliches Zusammensetzen der Vielzahl von Ultraschallabbildungen basierend auf der Vielzahl von Maskenabbildungen, um ein räumliches, zusammengesetztes Ultraschallbild zu erzeugen,
wobei e) Folgendes aufweist:
Summieren von Pixelwerten von Pixeln, die an den Ultraschallabbildungen überlappen, um einen ersten Additionswert zu erlangen;
Summieren von Pixelwerten von Pixeln, die an den Maskenabbildungen überlappen, um einen zweiten Additionswert zu erlangen; und
Teilen des ersten Additionswerts durch den zweiten Additionswert, um einen Pixelwert an einem korrespondierenden Pixel der räumlichen zusammengesetzten Ultraschallabbildung zu ermitteln.

5. Verfahren nach Anspruch 4, wobei die Masken identische Größen und Pixelanzahlen wie diejenigen der entsprechenden Ultraschallabbildungen aufweisen.

6. Verfahren nach Anspruch 4, wobei c) Folgendes aufweist:
Detektieren einer Intensität von jedem der Rahmen, die den jeweiligen Scanlinien entsprechen, unter Verwendung der Ultraschallrahmendatensätze;
Vergleichen der detektierten Intensität mit einem vorbestimmten Grenzwert, wenn die Intensität gleich oder größer als ein vorbestimmter Grenzwert ist, Zuordnen eines Werts von 1 zu den Pixeln, die der Scanlinie entsprechen; und
wenn die Intensität geringer als ein vorbestimmter Grenzwert ist, Zuordnen eines Werts von 0 zu den Pixeln, welche der Scanlinie entsprechen.

7. Computerlesbares Speichermedium, welches Befehle speichert, die, wenn sie von einem Computer ausgeführt werden, bewirken, dass der Computer ein Verfahren zum Erzeugen einer räumlichen, zusammengesetzten Ultraschallabbildung basierend auf einer Vielzahl von Ultraschallrahmendatensätzen, die von einem Zielobjekt und an unterschiedlichen Steuerungswinkeln von Scanlinien in einem Ultraschallsystem (100) erlangt werden, ausführt, wobei das Verfahren Folgendes aufweist:
Erzeugen von Masken, welche den jeweiligen Rahmen entsprechen, basierend auf den Ultraschallrahmendatensätzen;
Ermitteln von Pixelwerten von Pixeln von jeder der Maske basierend auf Intensitäten der Ultraschallrahmendatensätze zum Entfernen von Randartefakten, um eine Vielzahl von Maskenabbildungen zu erzeugen, die den jeweiligen Rahmen entsprechen;
Erzeugen einer Vielzahl von Ultraschallabbildungen, welche der Vielzahl von Rahmen entsprechen, basierend auf der Vielzahl von Rahmendatensätzen; und
räumliches Zusammensetzen der Vielzahl von Ultraschallabbildungen basierend auf der Vielzahl von Maskenabbildungen, um ein räumliches, zusammengesetztes Ultraschallbild zu erzeugen,
wobei das Ermitteln der Pixelwerte von Pixeln von jeder der Maske Folgendes aufweist:
Detektieren einer Intensität von jedem der Rahmen, die den jeweiligen Scanlinien entsprechen, unter Verwendung der Ultraschallrahmendatensätze;
Vergleichen der detektierten Intensität mit einem vorbestimmten Grenzwert, wenn die Intensität gleich oder größer als ein vorbestimmter Grenzwert ist, Zuordnen eines Werts von 1 zu den Pixeln, die der Scanlinie entsprechen; und
wenn die Intensität geringer als ein vorbestimmter Grenzwert ist, Zuordnen eines Werts von 0 zu den Pixeln, welche der Scanlinie entsprechen,
wobei das räumliche Zusammensetzen der Vielzahl von Ultraschallabbildungen Folgendes aufweist:
Summieren von Pixelwerten von Pixeln, die an den Ultraschallabbildungen überlappen, um einen ersten Additionswert zu erhalten;
Summieren von Pixelwerten von Pixeln, die an den Maskenabbildungen überlappen, um einen zweiten Additionswert zu erhalten; und
Teilen des ersten Additionswerts durch den zweiten Additionswert, um einen Pixelwert an einem entsprechenden Pixel der räumlichen zusammengesetzten Ultraschallabbildung zu ermitteln.

## Revendications

1. Système à ultrasons (100), comprenant :
une unité d'acquisition de données ultrasonores (110) configurée pour transmettre des faisceaux ultrasonores à un objet cible, recevoir des échos ultrasonores réfléchis par l'objet cible et fournir une pluralité d'ensembles de données de trames ultrasonores pour une pluralité de trames, ladite pluralité de données de trames ultrasonores étant acquise au niveau de différents angles de changement de direction de lignes de balayage ; et
une unité de traitement (120) configurée pour former une pluralité d'images ultrasonores,
dans lequel l'unité de traitement (120) comprend :
une section de formation de masques configurée pour former des masques correspondant aux trames respectives sur la base des ensembles de données de trames ultrasonores,
une section de réglage de masque configurée pour déterminer des valeurs de pixel des pixels de chacun des masques sur la base des intensités des ensembles de données de trames ultrasonores,
une section de formation d'image configurée pour former une pluralité d'images de masques sur la base des masques et la pluralité d'images ultrasonores sur la base des ensembles de données de trames ultrasonores, et
une section de composition spatiale configurée pour composer spatialement la pluralité d'images ultrasonores sur la base de la pluralité d'images de masques afin de former une image de composition spatiale ultrasonore,
dans lequel la section de composition spatiale est configurée pour additionner des valeurs de pixel de pixels en chevauchement au niveau des images ultrasonores pour obtenir une première valeur de somme, additionner des valeurs de pixel des pixels en chevauchement au niveau des images de masques pour obtenir une seconde valeur de somme et diviser la première valeur de somme par la seconde valeur de somme pour déterminer une valeur de pixel au niveau d'un pixel correspondant de l'image de composition spatiale ultrasonore.

2. Système à ultrasons (100) selon la revendication 1, dans lequel les images de masques ont des tailles et des nombres de pixels identiques à ceux des images ultrasonores correspondantes.

3. Système à ultrasons (100) selon la revendication 1, dans lequel la section de réglage de masque est configurée pour détecter une intensité de chacune des trames correspondant aux fines de balayage respectives en utilisant les ensembles de données de trames ultrasonores, comparer l'intensité détectée avec un seuil prédéterminé, attribuer, lorsque l'intensité est égale ou supérieure à un seuil prédéterminé, une valeur de 1 à des pixels correspondant à la ligne de balayage, et attribuer, lorsque l'intensité est inférieure au seuil prédéterminé, une valeur de 0 à des pixels correspondant à la ligne de balayage.

4. Procédé de formation d'une image de composition spatiale ultrasonore dans un système à ultrasons (100), comprenant les étapes consistant à :
a) transmettre des faisceaux ultrasonores à un objet cible, recevoir des échos ultrasonores réfléchis par l'objet cible et fournir une pluralité d'ensembles de données de trames ultrasonores pour une pluralité de trames, ladite pluralité de données de trames ultrasonores étant acquise à différents angles de changement de direction de lignes de balayage ;
b) former des masques correspondant aux trames respectives sur la base des ensembles de données de trames ultrasonores ;
c) déterminer des valeurs de pixel de pixels de chacun des masques sur la base des intensités des ensembles de données de trames ultrasonores pour supprimer un artefact de couture afin de former une pluralité d'images de masques correspondant aux trames respectives ;
d) former une pluralité d'images ultrasonores correspondant à la pluralité de trames sur la base de la pluralité d'ensembles de données de trames ; et
e) effectuer une composition spatiale de la pluralité d'images ultrasonores sur la base de la pluralité d'images de masques pour former une image de composition spatiale ultrasonore,
dans lequel e) comprend :
additionner des valeurs de pixel des pixels en chevauchement au niveau des images ultrasonores pour obtenir une première valeur de somme ;
additionner des valeurs de pixel de pixels en chevauchement au niveau des images de masques pour obtenir une seconde valeur de somme ; et
diviser la première valeur de somme par la seconde valeur de somme pour déterminer une valeur de pixel au niveau d'un pixel correspondant de l'image de composition spatiale ultrasonore.

5. Procédé selon la revendication 4, dans lequel les masques ont des tailles et des nombres de pixels identiques à ceux des images échographiques correspondantes.

6. Procédé selon la revendication 4, dans lequel le c) comprend les étapes consistant à :
détecter une intensité de chacune des trames correspondant aux lignes de balayage respectives en utilisant les ensembles de données de trames ultrasonores ;
comparer l'intensité détectée à un seuil prédéterminé,
attribuer, lorsque l'intensité est égale ou supérieure à un seuil prédéterminé, une valeur de 1 à des pixels correspondant à la ligne de balayage ; et
attribuer, lorsque l'intensité est inférieure au seuil prédéterminé, une valeur de 0 à des pixels correspondant à la ligne de balayage.

7. Support de stockage lisible par ordinateur stockant des instructions qui, lorsqu'elles sont exécutées par un ordinateur, amènent l'ordinateur à fournir un procédé de composition spatiale d'images ultrasonores sur la base d'une pluralité d'ensembles de données de trames ultrasonores acquis à partir d'un objet cible et à différents angles de changement de direction de lignes de balayage dans un système à ultrasons (100), le procédé comprenant les étapes consistant à :
former des masques correspondant aux trames respectives sur la base des ensembles de données de trames ultrasonores ;
déterminer des valeurs de pixel de pixels de chacun des masques sur la base des intensités des ensembles de données de trames ultrasonores pour supprimer un artefact de couture afin de former une pluralité d'images de masques correspondant aux trames respectives ;
former une pluralité d'images ultrasonores correspondant à la pluralité de trames sur la base de la pluralité d'ensembles de données de trames ; et
effectuer une composition spatiale de la pluralité d'images ultrasonores sur la base de la pluralité d'images de masques pour former une image de composition spatiale ultrasonore,
dans lequel la détermination de valeurs de pixel de pixels de chacun des masques comprend les étapes consistant à :
détecter une intensité de chacune des trames correspondant aux lignes de balayage respectives en utilisant les ensembles de données de trames ultrasonores ;
comparer l'intensité détectée à un seuil prédéterminé,
attribuer, lorsque l'intensité est égale ou supérieure à un seuil prédéterminé, une valeur de 1 à des pixels correspondant à la ligne de balayage ; et
attribuer, lorsque l'intensité est inférieure au seuil prédéterminé, une valeur de 0 à des pixels correspondant à la ligne de balayage,
dans lequel la composition spatiale de la pluralité d'images ultrasonores comprend les étapes consistant à :
additionner des valeurs de pixel des pixels en chevauchement au niveau des images ultrasonores pour obtenir une première valeur de somme ; additionner des valeurs de pixel de pixels en chevauchement au niveau des images de masques pour obtenir une seconde valeur de somme ; et
diviser la première valeur de somme par la seconde valeur de somme pour déterminer une valeur de pixel au niveau d'un pixel correspondant de l'image de composition spatiale ultrasonore.
